(19) Europäisches Patentamt
European Patent Office
Office européen des brevets

(11) Publication number: **0 291 321 A2**

(12) **EUROPEAN PATENT APPLICATION**

(21) Application number: 88304343.2

(22) Date of filing: 13.05.88

(51) Int. Cl.4: C 12 Q 1/34
G 01 N 33/52

(30) Priority: 14.05.87 US 49878

(43) Date of publication of application:
17.11.88 Bulletin 88/46

(84) Designated Contracting States: DE FR GB

(71) Applicant: EASTMAN KODAK COMPANY
Patent Department 343 State Street
Rochester New York 14650 (US)

(72) Inventor: Detwiler, Richard Linn EASTMAN KODAK COMPANY
Patent Department 343 State Street
Rochester New York 14650 (US)

Ferris, Robert James EASTMAN KODAK COMPANY
Patent Department 343 State Street
Rochester New York 14650 (US)

Fricker, Robert Frank EASTMAN KODAK COMPANY
Patent Department 343 State Street
Rochester New York 14650 (US)

(74) Representative: Nunney, Ronald Frederick Adolphe et al
Kodak Limited Patent Department Headstone Drive
Harrow Middlesex HA1 4TY (GB)

(54) **Element and method for determination of creatinine or creatine.**

(57) An analytical element for determining creatinine or creatine is disclosed. The element reduces the interference of lidocaine metabolites. The element comprises an absorbent carrier material containing 200-1,200 I.U./m$^2$ of the enzyme creatinine amidohydrolase, 35,000-65,000 I.U./m$^2$ of the enzyme creatine amidinohydrolase and 2,000-3,500 I.U./m$^2$ of the enzyme sarcosine oxidase and a leuco dye which is capable of providing a detectable dye in the presence of hydrogen peroxide and a peroxidative substance,

said creatine amidinohydrolase being present in a manner such that it is substantially inert to said leuco dye; and said creatinine amidohydrolase being present in a rate limiting amount and all three of said enzymes are present at a pH of about 6.5 to 6.9.

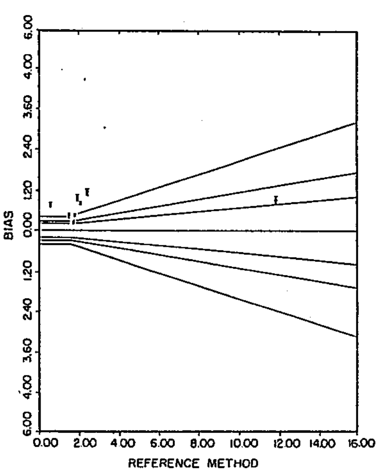


FIG. 2

EP 0 291 321 A2

**Description**

## ELEMENT AND METHOD FOR DETERMINATION OF CREATININE OR CREATINE

The present invention relates to clinical chemistry. In particular, it relates to an analytical element and method for the determination of either creatinine or creatine in aqueous liquids, e.g. biological fluids.

The determination of the intermediate and end products of protein metabolism is important in clinical chemistry, and particularly in the diagnosis of kidney function. The products of this metabolism include creatinine and creatine.

Enzymatic assays for creatinine have been developed using enzymes specific for creatinine and creatine, respectively, and the following reaction sequence:

(1) creatinine + water ⇄ creatine

(2) creatine + water → urea + sarcosine The first reaction is catalyzed by creatinine amidohydrolase whereas the second one is catalyzed by creatine amidinohydrolase.

In EP-A-0 243 066 published October 28, 1987, there is disclosed an analytical element for the determination of creatinine or creatine or both. The element contains 540 I.U./$m^2$ of creatinine amidohydrolase; 27,000 I.U./$m^2$ of creatine amidinohydrolase; 3,300 I.U./$m^2$ of sarcosine oxidase; and a leuco dye which is capable of providing a detectable dye in the presence of hydrogen peroxide and a peroxidative substance. The creatine amidinohydrolase is present in the manner such that it is substantially inert to the leuco dye. The creatinine amidohydrolase is present in a rate limiting amount. By measuring the amount of dye formation at particular times during the assay either creatinine or creatine or both can be determined with the same element.

Commercial embodiments of the above element contain 26,910 I.U./$m^2$ of creatine amidinohydrolase; 1,722 I.U./$m^2$ of sarcosine oxidase and 202 I.U./$m^2$ creatinine amidohydrolase. Use of this element has revealed that it exhibits a large clinically unacceptable bias in creatinine assay samples from patients being treated with lidocaine. It is believed that a metabolite of lidocaine (probably N-ethyl glycine) is interfering in the assays.

The problem encountered with the analytical element described in EP-A-0 243 066 is substantially overcome with an analytical element for the determination of either creatinine or creatine comprising an absorbent carrier material containing 200-1,200 I.U./$m^2$ of the enzyme creatinine amidohydrolase, 35,000-65,000 I.U./$m^2$ of the enzyme creatine amidinohydrolase and 2,000-3,500 I.U./$m^2$ of the enzyme sarcosine oxidase and a leuco dye which is capable of providing a detectable dye in the presence of hydrogen peroxide and a peroxidative substance,

said creatine amidinohydrolase being present in a manner such that it is substantially inert to said leuco dye; and said creatinine amidohydrolase being present in a rate limiting amount and all three of said enzymes are present at a pH of 6.5 to 6.9.

In a preferred embodiment, this invention provides a multilayer analytical element for the determination of either creatinine or creatine comprising a support having thereon, in order,

a first reagent layer containing 2,000-3,500 I.U./$m^2$ of the enzyme sarcosine oxidase, from 200-1,200 I.U./$m^2$ of the enzyme creatinine amidohydrolase, a peroxidative substance and an imidazole leuco dye which is capable of providing a detectable dye in the presence of said peroxidative substance and hydrogen peroxide,

a second reagent layer containing 35,000-65,000 I.U./$m^2$ of the enzyme creatine amidinohydrolase, and

a porous spreading layer wherein creatinine amidohydrolase is present in the first reagent layer in a rate limiting of amount, and all three of said enzymes are present in the element at a pH of 6.5 to 6.9.

The invention also provides a method for the determination of either creatinine or creatine comprising the steps of:

A. in the presence of a peroxidative substance, contacting a sample of a liquid suspected of containing either creatinine or creatine with an analytical element comprising an absorbent carrier material containing 200-1,200 I.U./$m^2$ of the enzyme creatinine amidohydrolase, 35,000-65,000 I.U./$m^2$ of the enzyme creatine amidinohydrolase and 2,000-3,500 I.U./$m^2$ of the enzyme sarcosine oxidase, and a leuco dye which is capable of providing a detectable dye in the presence of hydrogen peroxide and said peroxidative substance,

said creatine amidinohydrolase being present in a manner such that it is substantially inert to said leuco dye, and said creatinine amidohydrolase being present in a rate limiting amount and all three of said enzymes are present in the element at a pH of 6.5 to 6.9, and

B. determining the detectable dye formed as a result of the presence of either creatinine or creatine.

Through extensive testing, the present invention provides an improved analytical element in which the amounts of the various enzymes have been carefully selected within the broad range of amounts disclosed for use in such elements by the prior art. Moreover, the pH has been adjusted to minimize the specific interference of lidocaine metabolites on the activity of sarcosine oxidase.

The present invention provides a relatively simple, automated means to rapidly and economically measure either creatinine or creatine or both with the same analytical element. Separate analytical compositions or elements are therefore not needed for the different analytes. The assay of this invention is rapid, allowing measurement of either analyte within, for example, about 5 minutes. It is also possible to avoid tedious blanking steps with the present invention. The complex equipment used for solution assays described in Japanese Patent Publication 58(1983)-009699 is therefore avoided with the present invention.

These advantages are possible with the present invention because it is a kinetic assay for creatinine

performed with the analytical element described herein. This element contains creatinine amidohydrolase and creatine amidinohydrolase which promote the two enzymatic reactions (1) and (2) noted above. However, the creatinine amidohydrolase is present in a rate limiting amount so that once endogenous creatine has completely reacted, the rate of dye formation is due totally to the presence of creatinine. However, creatine can also be accurately measured with this element by measuring the amount of dye formation early in the assay. Background is minimized by putting the reagents into the element in such a manner that they do not interfere with each other. In particular, the creatine amidinohydrolase is present in a manner such that it is substantially inert to the leuco dye.

FIG. 1 is a graphical plot of bias distribution in creatinine assays of patients on lidocaine therapy using a prior art element versus a reference method.

FIG. 2 is a graphical plot of bias distribution in creatinine assays of patients on lidocaine therapy using an element of this invention versus a reference method.

The present invention relates to the determination (qualitative or quantitative measurement) of either creatinine or creatine in aqueous liquids. In particular, the invention can be used to assay biological fluids of animals and humans. Such fluids include, but are not limited to, whole blood, plasma, sera, lymph, bile, urine, spinal fluid, sputum, perspiration and the like as well as stool secretions. It is also possible to assay fluid preparations of human or animal tissue such as skeletal muscle, heart, kidney, lungs, brains, bone marrow, skin and the like. Preferably, human serum or urine is assayed with this invention.

The method of this invention is practiced using a dry analytical element. The simplest element can be composed of an absorbent carrier material, e.g. a thin sheet of a self-supporting absorbent or bibulous material, such as filter paper or strips, which contains the reagents needed for the assay. The element can be divided into two or more discrete zones with different reagents incorporated into individual zones of the carrier material. Such elements are known in the art as test strips, diagnostic elements, dip sticks, diagnostic agents and the like.

Useful absorbent carrier materials are insoluble and maintain their structural integrity when exposed to water or biological fluids such as urine or serum. Useful elements can be prepared from paper, porous particulate structures, porous polymeric films, cellulose, glass fibers, woven and nonwoven fabrics (synthetic and nonsynthetic) and the like. Useful materials and procedures for making such elements are well known in the art.

Preferably, the dry analytical element of this invention has a porous spreading zone. This zone can be self-supporting (i.e. composed of a material rigid enough to maintain its integrity), but preferably it is carried on a separate nonporous support. Such a support can be any suitable dimensionally stable, and preferably, nonporous and transparent (i.e. radiation transmissive) material which transmits electromagnetic radiation of a wavelength between 200 and 900 nm. A support of choice for a particular element should be compatible with the intended mode of detection (transmission or reflectance spectroscopy). Useful supports can be prepared from paper, metal foils, polystyrene, polyesters [e.g. poly(ethylene terephthalate)], polycarbonates, cellulose esters (e.g. cellulose acetate), etc.

The porous spreading zone can be prepared from any suitable fibrous or non-fibrous material or mixtures of either or both as described in U.S. Patents 4,292,272; 3,992,158; 4,258,001; 430,436 and Japanese Patent Publication 57(1982)-101760 (published June 24, 1982). It is desirable that the spreading zone be isotropically porous, meaning that the porosity is the same in each direction in the zone as caused by interconnected spaces or pores between particles, fibers, polymeric strands, etc.

The elements can have two or more discrete zones, either in the same layer or superimposed, at least one of which is preferably a porous spreading zone. The other zones can be reagent zones or registration zones as those zones are known in the art, additional spreading zones, radiation-blocking or filter zones, subbing zones, barrier zones, etc. The zones are generally in fluid contact with each other, meaning that generally fluids, reagents and reaction products (e.g. color dyes) can pass or be transported between superposed regions of adjacent zones. Preferably, the zones are separately coated and superposed layers.

The assay of this invention is accomplished with the following sequence of reactions (1)-(4):

(1) creatine + water ⇌ creatine
(2) creatine + water → urea + sarcosine
(3) sarcosine + oxygen + water → glycine + formaldehyde + hydrogen peroxide
(4) hydrogen peroxide + leuco dye → detectable dye.

These reactions are catalyzed by creatinine amidohydrolase, creatine amidinohydrolase, sarcosine oxidase and a peroxidative substance, respectively.

The enzymes described herein can be used in the practice of this invention in pure form, as fermentation solutions or as impure extracts of the enzymes, individually or collectively.

Creatinine amidohydrolase and creatine amidinohydrolase can be obtained commercially from a number of sources. Several species of each enzyme, isolated from various microbial sources, are described in U.S. Patents 3,806,416 and 4,039,384. Any of the species can be used in the practice of this invention. The creatinine amidohydrolase and the creatine amidinohydrolase, both obtained from a strain of Flavobacterium and described in U.S. Patent 4,039,384, are useful.

Sarcosine oxidase can also be obtained commercially from a number of sources. Sarcosine oxidase from Bacillus species is useful in the practice of this invention.

Peroxidative substances useful in this invention includes peroxidase. A peroxidase is an enzyme which will

catalyze a reaction wherein hydrogen peroxide oxidizes another substance. The peroxidases are generally conjugated proteins containing iron porphyrin. Peroxidase occurs in horseradish, potatoes, fig tree sap and turnips, milk and while blood cells. It also occurs in microorganisms and can be produced by fermentation. Certain synthetic peroxidases are also known. Peroxidase is a preferred peroxidative substance, but other substances which are not enzymes are also useful. Many of these are commercially available.

The leuco dyes useful in this invention are imidazole leuco dyes which are generally colorless in the leuco form, but which can be oxidized to a detectable colored dye in the presence of hydrogen peroxide and a peroxidative substance. Useful leuco dyes include di- and triarylimidazoles such as those described in U.S. Patent 4,089,747, E.P. Application 122,641 and Jap. Patent Publication 58(1983)-045,557. Particularly useful imidazole leuco dyes are the triarylimidazoles described in U.S. Patent 4,089,747, including, e.g. 2-(3,5-dimethoxy-4-hydroxyphenyl)-4,5-bis(4-dimethyl-aminophenyl)imidazole, 2-(4-hydroxy-3-methoxyphenyl)-4,5-bi(p-dimethylaminophenyl)-1H-imidazole, 2-(3-ethoxy-4-hydroxyphenyl-4,5-bis(p-dimethylaminophenyl)-1H-imidazole, 2-(4-hydroxy-3,5-dimethoxyphenyl)-4-[4-(dimethylamino)-phenyl]-5-(2-furyl)imidazole, 2-(4-hydroxy-3,5-dimethoxyphenyl)-4,5-di(2-furyl)imidazole, 2-(3,5-dimethoxy4-hydroxyphenyl)-4-[4-(dimethylamino)phenyl]-5-phenethylimidazole and 2-(3,5-dimethoxy-4-hydroxy-phenyl)-4-[4-(dimethylamino)phenyl]-5-benzylimidazole.

The elements of this invention can also contain one or more other addenda commonly included in the elements for various manufacturing or operational advantages. Such addenda include surfactants, ion chelating agents, buffers, solvents, hardeners, antioxidants, coupler solvents, and the like.

It is critical that the creatinine amidohydrolase be present in the element in a rate limiting amount. This means that the amount of this enzyme in relation to the amount of creatine amidinohydrolase is such that the forward direction of reaction (1) noted above is rate controlling. The specific amount of creatinine amidohydrolase can be readily determined by a skilled clinical chemist. Generally, the amount is preferably from 200 to 1,200, I.U./$m^2$. Creatine amidinohydrolase can be present in any amount as long as it is not a rate limiting amount. In other words, reaction (2), noted above, is not to be controlling in the assay. The specific amount of this enzyme can be readily determined by a skilled clinical chemist.

As used in the context of this disclosure and the claims, I.U. represents the International Unit for enzyme activity defined as one I.U. being the amount of enzyme activity required to catalyze the conversion of 1 micromole of substrate per minute under standard pH and temperature conditions for the enzyme. For the preferred enzyme preparations used in this invention, these standard conditions are 30° C and pH 8.0 for creatinine amidohydrolase, 37° C and pH 7.4 for creatine amidinohydrolase, 37° C and pH 7.4 for sarcosine oxidase and 25° C and pH 7.0 for peroxidase.

The other reagents useful in the assay are present in suitable amounts readily determined by a skilled clinical chemist. Representative amounts are illustrated in the examples below.

It is critical in the practice of this invention that the creatine amidinohydrolase and leuco dye are present in a manner such that the enzyme is substantially inert to the dye. This means that the two reagents are incorporated in the element in such a manner that the enzyme does not adversely affect the leuco dye. This can be accomplished in a number of ways. For example, the enzyme can be used in a highly pure form so that the leuco dye is not affected by any impurities. More practically, however, the enzyme cannot be obtained in a highly pure form. In such cases, either or both the enzyme and dye can be encapsulated or otherwise isolated from each other in the element until the assay is carried out. Preferably, the enzyme and leuco dye are located in different zones or layers of the element so that they do not mix until the time of the assay.

A variety of different elements, depending on the method of assay, can be prepared in accordance with the present invention. Elements can be configured in a variety of forms, including elongated tapes of any desired width, sheets, slides or chips.

The assay of this invention can be manual or automated. In general, in using the dry elements, creatinine or creatine determination is made by taking the element from a supply roll, chip packet or other source and physically contacting it with a sample (e.g. 1 to 200 µl) of the liquid to be tested so that the sample and reagents within the element become mixed. Such contact can be accomplished in any suitable manner, e.g. dipping or immersing the element into the sample or, preferably, by spotting the element by hand or machine with a drop of the sample with a suitable dispensing means.

After sample application, the element is exposed to any conditioning, such as incubation, heating or the like, that may be desirable to quicken or otherwise facilitate obtaining any test result.

The rate of dye formation is then measured with suitable reflection or transmission spectrophotometric equipment and procedures. Generally, for creatine determination, a dye measurement is made prior to substantial conversion of creatinine, i.e. soon after sample-element contact, e.g. prior to about 1 minute after sample-element contact. This measurement determines endogenous creatine because the rate limiting amount of creatinine amidohydrolase in the element has converted substantially no creatinine to creatine at this point.

For creatinine determination, at least two dye measurements are made after substantially all endogenous creatine has been converted enzymatically to reaction products. Generally, the first measurement is made about 4 minutes after sample-element contact. Another measurement is made thereafter in order to determine the rate of dye formation, and hence, the amount of creatinine. This sequence of measurements allows the use of the present element to measure either or both creatinine and creatine.

As stated hereinbefore, a metabolite of lidocaine, believed to be N-ethyl glycine, sometimes creates

clinically unacceptable bias when the commercial embodiments of the aforementioned EP-A-0 243 066 are used to assay creatinine. The belief that N-ethyl glycine is the interferent is supported by the similarities in bias observed in patient samples and samples containing known amounts of N-ethyl glycine.

The following example 1 shows how pH of the element of this invention decreases the effect of N-ethyl glycine as an interferent on the activity of the enzyme sarcosine oxidase.

Example 1

Separate assays at various pH were conducted to determine the rate of change in absorbance per minute (ΔA/min) for sarcosine oxidase activity on its substrate sarcosine, and on N-ethyl glycine. The assay is based on the following reactions.

$$\text{substrate (sarcosine or N-ethyl glycine)} + O_2 \xrightarrow[\text{SARCOSINE OXIDASE}]{} H_2O_2 + \text{glycine} + \text{formaldehyde or acetaldehyde}$$

$$H_2O_2 + \text{hydrogen donor} \xrightarrow[\text{PEROXIDASE}]{} \text{oxidized donor (chromophore)} + 2H_2O$$

Rate of formation of a chromophore due to oxidation of the donor (4-aminoantipyrine) is monitored at 480 nm spectrophotometrically.

The following data were obtained.

TABLE I

| | A Sarcosine | B N-Ethyl Glycine | Ratio (B/A) |
|---|---|---|---|
| pH 7.5 (ΔA/min) | 2.76 | 0.134 | 0.048 |
| pH 7.0 (ΔA/min) | 2.20 | 0.053 | 0.024 |
| pH 6.5 (ΔA/min) | 1.56 | 0.017 | 0.011 |

Ideally, the rate with sarcosine should be high, and the rate from N-ethyl glycine should be zero. So the lower the ratio of the rate of N-ethyl glycine to that of sarcosine, the better.

Table I shows that the N-ethyl glycine/sarcosine ratio decreases significantly as the pH is lowered. This shows that sarcosine oxidase is functioning as a more specific enzyme at the lower pH. A pH of 6.5 to 6.9 was chosen for the element of this invention to optimize and balance the performance of all the enzymes.

In the example which follows, illustrating the practice of this invention, the materials used were obtained as follows:

TRITON X-100 and X-405 surfactants from Rohm and Haas (Philadelphia, Pennsylvania, U.S.A.);
BRIJ 78 surfactant from ICI Americas (Wilmington, Delaware, U.S.A.);
ESTANE polyurethane resin from B. F. Goodrich (Cleveland, Ohio, U.S.A.);
ALKANOL XC surfactant from DuPont (Wilmington, Delaware, U.S.A.);
creatinine amidohydrolase and creatine amidinohydrolase from Seishin Pharmaceutical (Japan); and sarcosine oxidase from Toyo Jozo Company Ltd. (Japan);
peroxidase from Miles Laboratories (Elkhart, Indiana, U.S.A.);
N-tris-(hydroxymethyl)methyl-2-aminomethane sulfonic acid buffer from Sigma Chemicals (St. Louis, Missouri, U.S.A.);
and the remainder either from Eastman Kodak Company (Rochester, New York, U.S.A.) or prepared using standard procedures and readily available starting materials.

Example 2 Assays for Creatine and Creatinine

This example illustrates the practice of this invention for the determination of creatinine. This assay was carried out with the element having the following components. Except as otherwise noted, suitable ranges of quantities of each component are shown in parenthesis.

| | | |
|---|---|---|
| Spreading Layer | Titanium dioxide | 50 (20–80) $g/m^2$ |
| | Cellulose acetate | 7 (2–10) $g/m^2$ |
| | BRIJ 78 surfactant | 0.8 (0.3–1.5) $g/m^2$ |
| | TRITON X-405 surfactant | 1.7(0.5–5) $g/m^2$ |
| | ESTANE resin | 2 (1–5) $g/m^2$ |
| Subbing Layer | Poly(N-isopropylacrylamide) | 0.4 (0.1–1) $g/m^2$ |

| | | |
|---|---|---|
| Reagent Layer | Gelatin | 10.8 (1–20)g/$m^2$ |
| | N–tris–(hydroxymethyl)–methyl–2–aminomethane sulfonic acid buffer | 2.7 (0.5–5)g/$m^2$ |
| | (Ethylenedinitrilo)tetra–acetic acid | 0.3 (0.1–1)g/$m^2$ |
| | Creatine amidinohydrolase at pH of 6.5 to 6.9 at 40°C | 40,350 (35,000–65,000)I.U./$m^2$ |
| | Ascorbic acid oxidase | 5,400 (1,000–10,000) I.U./$m^2$ |
| | TRITON X–100 surfactant | 0.5 (0.1–2.5)g/$m^2$ |
| Registra–tion/Reagent Layer | Gelatin (hardened) | 6.5 (1–20)g/$m^2$ |
| | Sarcosine oxidase at pH of 6.5 to 6.9 at 40°C | 2,900 (2,000–3,500)I.U./$m^2$ |
| | Peroxidase | 33,000 (500–80,000)I.U./$m^2$ |
| | 5,5–dimethyl–1,3–cyclo–hexanedione | 0.09 (0.01–1)g/$m^2$ |
| | 2,4–di–*n*–pentyl phenol | 3.2 (0.5–5)g/$m^2$ |
| | Creatinine amidohydrolase at a pH of 6.5 to 6.9 at 40°C | 809 (200–1,200)I.U./$m^2$ |
| | N–tris–(hydroxymethyl)–methyl–2–aminomethane sulfonic acid buffer | 2.7 (0.5–5)g/$m^2$ |
| | (Ethylenedinitrilo)tetra–acetic acid | 0.32 (0.1–1)g/$m^2$ |
| | TRITON X–100 surfactant | 0.5 (0.1–2.5)g/$m^2$ |
| | ALKANOL XC surfactant | 0.5 (0.1–2.5)g/$m^2$ |
| | 2–(3,5–dimethoxy–4–hydroxyphenyl)–4,5–bis(4–methylamino–phenyl)imidazole | 0.32 (0.1–1)g/$m^2$ |

Poly(ethylene terephthalate) Support

This element was used to determine creatinine in the following manner. A calibration curve was prepared for creatinine by applying 10 µl samples of the appropriate calibrator fluids to separate elements, incubating and measuring the resulting dye at 670 nm. Reflectance density ($D_R$) readings were made at 231 and 300 seconds after addition of each sample. A calibration curve for creatinine was obtained by subtracting the reading at 231 seconds from the respective reading taken at 300 seconds and dividing by 1.217 for each calibrator sample. This curve enables one to determine the kinetic rate of reaction for creatinine.

Creatinine was then measured in 31 different patient samples containing unknown amounts of this analyte. Many of these patients were known to be on high levels of lidocaine therapy. Each sample was measured in 1) an element of this invention and 2) the previously mentioned commercial embodiment of the element disclosed in EP-A-0 243 066. The measurement was made by applying a sample of the test fluid to the element, reading the reflectance densities at the appropriate times and determining the respective analyte concentration from the appropriate calibration curve. The bias calculated against the results of a reference method for each of the 31 samples and for each element were plotted. Figure 1 shows bias distribution against the reference for the commercial element. Figure 2 shows the bias distribution against the reference for the element of the invention. A comparison of the two figures shows that the bias distribution is much greater for the commercial element than for the element of this invention. The overall bias distribution of the element of this invention is more closely focused along the zero bias line of the figures than the distribution of the commercial element.

**Claims**

1. An analytical element for the determination of either creatinine or creatine comprising an absorbent carrier material containing 200-1,200 I.U./$m^2$ of the enzyme creatinine amidohydrolase, 35,000-65,000 I.U./$m^2$ of the enzyme creatine amidinohydrolase and 2,000-3,500 I.U./$m^2$ of the enzyme sarcosine oxidase and a leuco dye which is capable of providing a detectable dye in the presence of hydrogen peroxide and a peroxidative substance,

said creatine amidinohydrolase being present in a manner such that it is substantially inert to said leuco dye; and said creatinine amidohydrolase being present in a rate limiting amount and all three of said enzymes are present at a pH of about 6.5 to 6.9.

2. The element of claim 1 wherein said creatine amidinohydrolase and leuco dye are physically located in separate zones of said element.

3. The element of claim 1 wherein said leuco dye is a triarylimidazole leuco dye.

4. The element of claim 1 further comprising a peroxidative substance.

5. The element of claim 1,2,3 or 4 comprising a support having thereon, in order,

a first reagent layer containing 2,000-3,500 I.U./$m^2$ of the enzyme sarcosine oxidase, from 200-1,200 I.U./$m^2$ of the enzyme creatinine amidohydrolase, a peroxidative substance and an imidazole leuco dye which is capable of providing a detectable dye in the presence of said peroxidative substance and hydrogen peroxide,

a second reagent layer containing 35,000-65,000 I.U./$m^2$ of the enzyme creatine amidinohydrolase, and

a porous spreading layer wherein creatinine amidohydrolase is present in the first reagent layer in a rate limiting amount, and all three of said enzymes are present in the element at a pH of 6.5 to 6.9.

6. A method for the determination of either creatinine or creatine comprising the steps of:

A. in the presence of a peroxidative substance, contacting a sample of a liquid suspected of containing either creatinine or creatine with an analytical element comprising an absorbent carrier material containing 200-1,200 I.U./$m^2$ of the enzyme creatinine amidohydrolase, 35,000-65,000 I.U./$m^2$ of the enzyme creatine amidinohydrolase and 2,000-3,500 I.U./$m^2$ of the enzyme sarcosine oxidase, and a leuco dye which is capable of providing a detectable dye in the presence of hydrogen peroxide and said peroxidative substance,

said creatine amidinohydrolase being present in a manner such that it is substantially inert to said leuco dye, and said creatinine amidohydrolase being present in a rate limiting amount and all three of said enzymes are present in the element at a pH of about 6.5 to 6.9, and

B. determining the detectable dye formed as a result of the presence of either creatinine or

creatine.

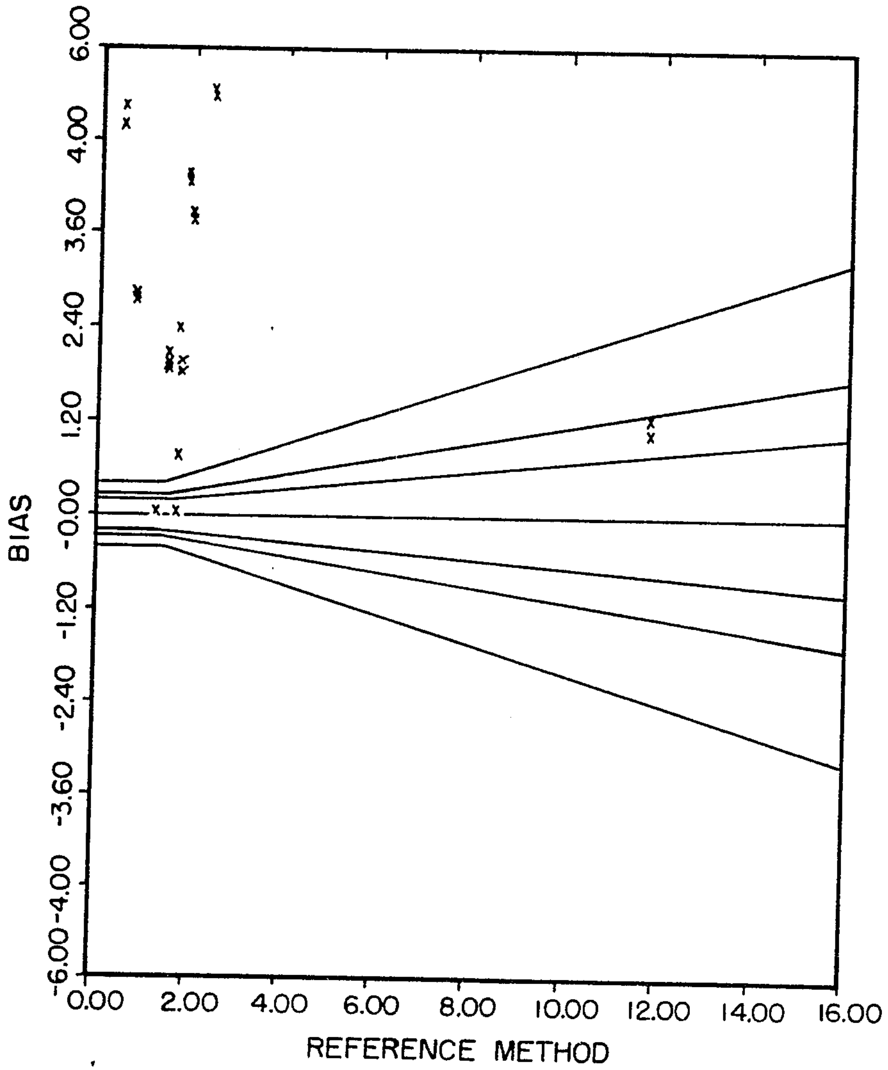
6.00
4.00
3.60
2.40
1.20
-0.00
-1.20
-2.40
-3.60
-6.00-4.00
BIAS
0.00
2.00
4.00
6.00
8.00
10.00
12.00
14.00
16.00
REFERENCE METHOD

FIG. 1

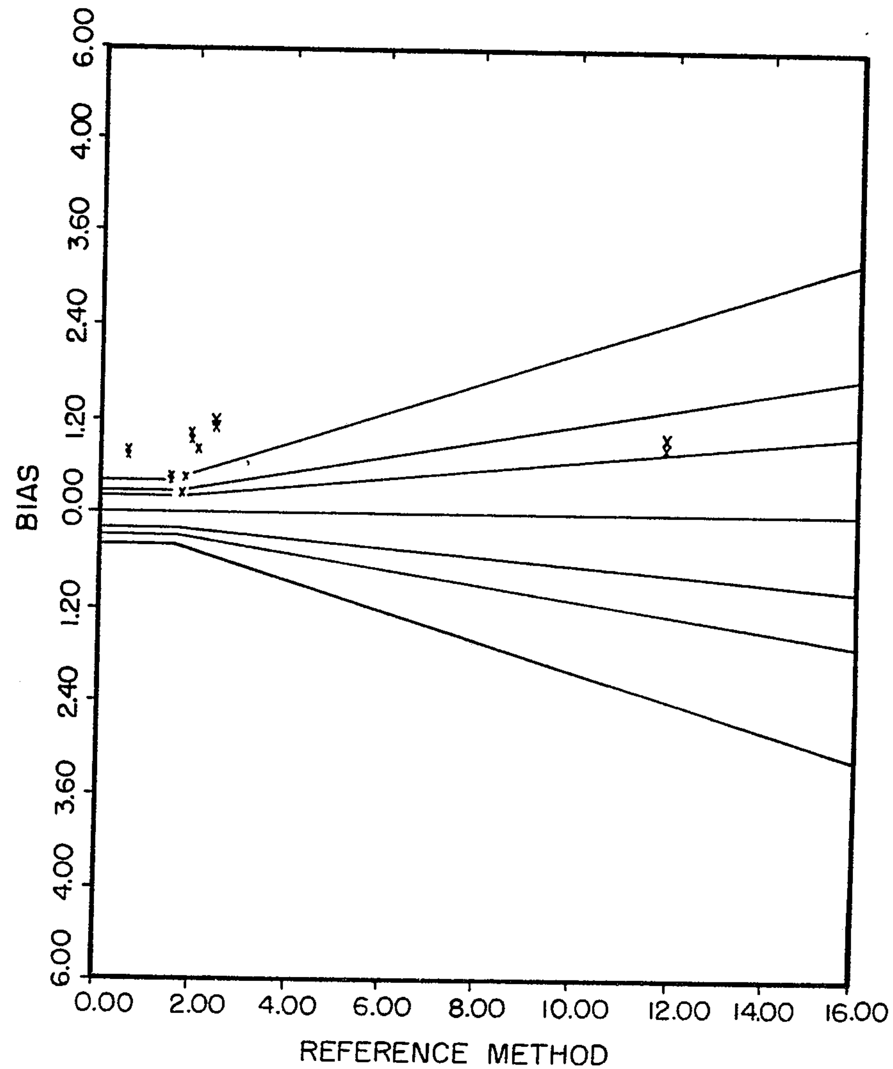
6.00
4.00
3.60
2.40
1.20
0.00
1.20
2.40
3.60
4.00
6.00
BIAS
0.00
2.00
4.00
6.00
8.00
10.00
12.00
14.00
16.00
REFERENCE METHOD

FIG. 2